# FASCICULE DE BREVET EUROPEEN

(11) **EP 0 322 260 B1**
(45) Date de publication et mention de la délivrance du brevet: **14.09.1994**
(21) Numéro de dépôt: 88402831.7
(22) Date de dépôt: 10.11.1988
(51) Int. Cl.: A61B 6/00

(54) **Dispositif de mammographie**
Mammographisches Gerät
Mammographic apparatus

(30) Priorité: 17.11.1987 FR 8715844
(43) Date de publication de la demande: 28.06.1989
(73) Titulaire: GE MEDICAL SYSTEMS S.A., F-92130 Issy les Moulineaux (FR)
(72) Inventeur: Peyret, Olivier, F-75116 Paris (FR); Delair, Jacques, F-75116 Paris (FR)
(74) Mandataire: Ballot, Paul Denis Jacques

(56) Documents cités:
- WO-A-87/01555
- FR-A- 2 208 298
- MEDICAL PHYSICS vol.8, no.5, Septembre/Octobre 1981, pages 629-639, New York US; R.J.Jennings et al: "Optimal X-Ray spectra for screen-film mammography"

## Description

L'invention concerne un dispositif de mammographie, particulièrement des moyens pour améliorer la qualité d'images obtenues par des radiographies d'un sein ayant une densité et/ou une épaisseur supérieure à la moyenne.

Avec un tube à rayons X, le rayonnement X est obtenu sous l'effet d'un bombardement électronique d'une anode, ou plus précisément d'une cible ; la cible peut être rapportée sur l'anode, ou être directement formée par l'anode elle-même qui dans ce cas est réalisée dans le matériau destiné à constituer la cible. Sur la cible, une faible surface est soumise au bombardement électronique et constitue la source d'un rayonnement X. Certaines des caractéristiques du rayonnement X dépendent des caractéristiques du faisceau d'électrons incidents, et de la nature du matériau dont est constituée la cible.

Ainsi par exemple pour la mammographie, il est courant d'utiliser des cibles en molybdène. L'intérêt d'une cible en molybdène en mammographie, réside notamment dans le fait que le spectre d'énergie du rayonnement X émis convient particulièrement bien à la spécificité d'un examen radiologique du sein. En effet le sein a une absorption au rayonnement X faible, et les anomalies recherchées ont des densités très proches de celles des tissus environnants. Le contraste présenté sur une radiographie, entre ces anomalies et les tissus environnants, est amélioré de manière importante quand le spectre du rayonnement X utilisé présente une bande en énergie relativement étroite, contenant notamment les raies caractéristiques (K alpha, K béta) de molybdène. Un tel spectre de rayonnement X est obtenu de manière classique, en bombardant par un faisceau d'électrons une cible en molybdène pour produire un rayonnement X qui est filtré à l'aide d'un filtre en molybdène, et aussi en conférant aux électrons incidents une énergie suffisamment plus élevée que l'énergie de liaison des électrons de la couche en réglant la valeur de la haute tension appliquée entre la cathode et l'anode du tube à rayons X.

Cependant il existe une dispersion importante des caractéristiques de transmission du rayonnement X par le sein. Ceci est dû notamment aux variations importantes de taille et de composition du sein d'une femme à une autre.

Ainsi le rayonnement X produit par le molybdène, et filtré comme il a été ci-dessus expliqué, est particulièrement bien adapté à l'examen radiologique de seins de composition moyenne et de taille moyenne par exemple ou encore de taille moyenne et de composition peu dense à moyenne. Par contre l'utilisation d'un tel spectre de rayonnement X pour la radiographie de seins de taille importante et/ou de composition plus dense présente un problème, du fait que l'absorption du rayonnement X est nettement plus importante que dans le premier cas.

Ainsi par exemple on observe que pour un sein de composition moyenne ayant une épaisseur de 5cm, environ 2 % du rayonnement X ressort du sein et peut impressioner le film radiographique, alors que dans le cas d'un sein d'une épaisseur d'environ 7cm, seulement 0,2 % environ du rayonnement X sort du sein après avoir traversé ce dernier, et par suite le film est insuffisamment impressionné.

La solution classique à ce problème consiste à augmenter le temps de pose jusqu'à obtenir une image correcte sur le film. Mais cette solution présente notamment les deux inconvénients très importants suivants :
- premièrement on observe une détérioration de la qualité d'image qui s'explique par une augmentation du flou cinétique ou flou de bougé ;
- deuxièmement l'augmentation du temps de pose conduit à une augmentation de la dose absorbée par la patiente, cette augmentation de dose pouvant être très importante par suite de l'écart à la loi de réciprocité du couple film/écran utilisé en mammographie : en effet, cet écart à la loi de réciprocité du couple film/écran ou récepteur, fait que la quantité de photons, nécessaire au même noircissement du film, croît lorsque les temps d'exposition augmentent.

Ainsi pour les seins de tailles plus importantes et/ou de composition plus dense, non seulement on perd l'avantage de la qualité d'image apportée par le rayonnement produit à l'aide d'un matériau cible en molybdène, mais en outre on augmente de manière non négligeable la dose de rayonnement X absorbée par la patiente.

FR-A-2 208 298 montre un dispositif de mammographie à double foyer qui permet par rotation de la patiente de passer de l'observation du sein gauche à celle du sein droit.

L'invention concerne un dispositif de mammographie dont le tube radiogène comporte une anode agencée de manière à permettre notamment une radiographie des seins les plus épais et/ou les plus denses, avec une qualité d'image au moins égale à celle obtenue avec un rayonnement X produit à partir d'une cible en molybdène et filtrée avec du molybdène, et ceci avec des temps de pose comparables à ceux qui sont utilisés dans le cas des seins plus petits ou moins denses.

Selon l'invention, un dispositif de mammographie comportant un tube radiogène (6) produisant un faisceau de rayons X (95), le tube radiogène comportant une anode (9) et des moyens (8,30,31) pour produire au moins un faisceau d'électrons 33), est caractérisé en ce que:
- l'anode du tube comporte deux cibles, l'une en rhodium (21), l'autre en molybdène(20), le rayonnement X produit à partire de la première ou la deuxième cible étant émis dans toutes les directions et sortant du tube par des fenêtres orientées vers un plateau d'examen (85);
- en ce qu'il comporte un dispositif de filtration (89) muni d'un diaphragme pour le passage du faisceau de rayons X, un filtre (103) en rhodium ou en un matériau ayant un numéro atomique proche du rhodium interposé sur le trajet (96) du faisceau de rayons X émis par la cible en rhodium et un filtre en molybdène (102) interposé sur le trajet (96) du faisceau de rayons (X) émis par la cible en molybdène, ces filtres pouvant être placés alternativement au dessus du diaphragme en fonction de la cible qui émet; et
- en ce que l'ensemble tube et dispositif de filtration étant porté par un bras (3), le tube étant incliné par rapport au dispositif de filtration de manière à ce que le rayonnement utilisé soit pris dans les limites (92,93) du faisceau définies par le diaphragme et de manière à ce que le maximum d'intensité du rayonnement se trouve autour d'un seul axe (96), quelle que soit la cible qui émet.

Il est possible à partir d'une cible en rhodium d'obtenir un rayonnement X qui, après filtrage par un filtre en rhodium, présente un spectre en énergie relativement étroit comprenant les raies caractéristiques K alpha et K béta qui, pour le rhodium sont situées respectivement à environ 20,2 KeV et 22,7 KeV, l'énergie moyenne du rayonnement X émis étant de l'ordre de 20 KeV; alors que pour le rayonnement produit à partir d'une cible en molybdène et filtré par du molybdène, l'énergie moyenne est de l'ordre de 17 KeV. Il en résulte que pour l'examen d'un sein d'épaisseur ou de densité plus grande que la moyenne, le rayonnement X provenant du rhodium et filtré par le rhodium, permet de réaliser une image avec un même noircissement du film et pour un même temps de pose que dans le cas du rayonnement X produit à partir du molybdène et filtré par du molybdène, pour l'examen d'un sein d'épaisseur ou de densité plus faible.

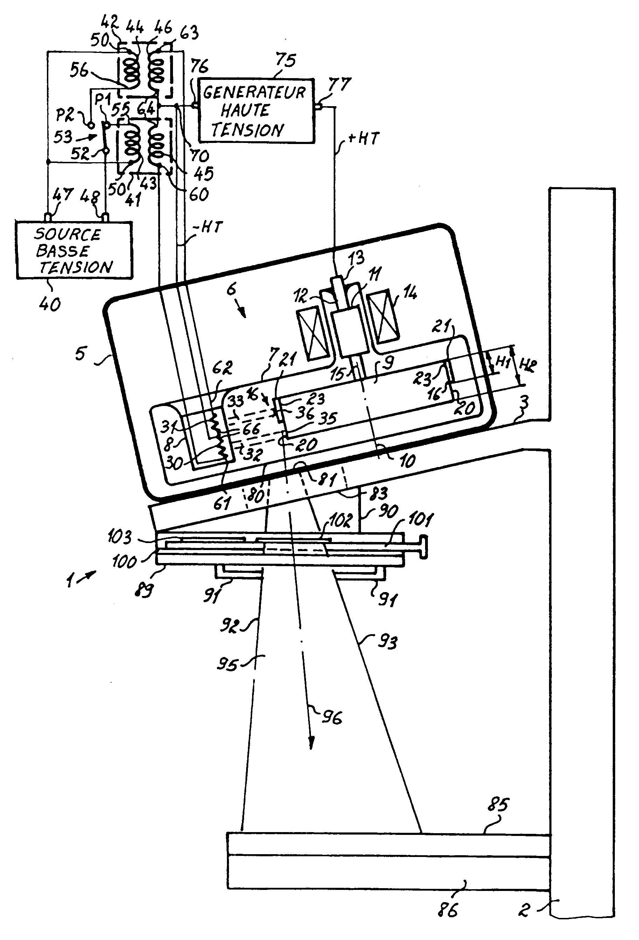

L'invention sera mieux comprise grâce à la description qui suit, faite à titre d'exemple non limitatif, et à l'unique figure annexée qui montre schématiquement un dispositif de mammographie dans une version préférée de l'invention.

La figure unique montre de manière schématique et à titre d'exemple non limitatif, un dispositif de mammographie 1 conforme à l'invention. Le dispositif de mammographie comporte un statif 2 (partiellement représenté) dont un bras 3 porte une gaîne 5 contenant un tube à rayons X 6. Le tube à rayons X 6 comporte une enveloppe 7 étanche au vide dans laquelle sont disposées une cathode 8 et une anode 9.

Dans l'exemple non limitatif décrit, l'anode 9 a la forme d'un disque et constitue une anode tournante dont la rotation s'effectue autour d'un axe de symétrie 10 du disque d'anode, à l'aide d'un rotor 11. Le rotor 11 est solidarisé au disque d'anode 9 par un axe support 15, et le rotor est porté d'autre part par un arbre support 12 métallique dont une extrémité 13 sort de l'enveloppe 7, l'arbre support 12 étant lui-même porté par l'enveloppe 7 ; la rotation du rotor 11 s'effectue sous l'action d'un stator 14 extérieur à l'enveloppe 13.

Dans l'exemple non limitatif de la description, la cathode 8 est portée en vis-à-vis de la tranche ou pourtour 16 du disque d'anode 9, de sorte que c'est la tranche 16 qui est destinée à constituer ou à porter au moins une cible qui, sous l'effet d'un bombardement électronique, produit un rayonnement X approprié à la radiographie d'un sein (non représenté). Selon une version préférée de l'invention, la tranche 16 de l'anode 9 comporte deux cibles 20, 21 de nature différente : la première cible 20 est en molybdène et, selon une caractéristique de l'invention, la seconde cible 21 est en rhodium.

Dans l'exemple non limitatif décrit, la première cible 20 en molybdène est formée par l'anode 9 elle-même qui est réalisée directement en molybdène, par frittage de poudre de molybdène par exemple ; et dans ces conditions, la seconde cible 21 rhodium est constituée par une couche 23 de rhodium déposée sur la tranche 16 à l'aide d'un procédé classique, tel que par exemple par dépôt électrolytique, par pulvérisation cathodique, ou encore par dépôt chimique en phase vapeur (CVD). La couche de rhodium 23 est déposée sur la tranche 16 c'est-à-dire sur tout le tour du disque d'anode 9, de sorte à constituer une piste focale. Dans l'exemple non limitatif décrit, la couche 23 de rhodium est déposée sur la tranche 16, sur une hauteur HI qui représente sensiblement la moitié de la hauteur H2 de la tranche 16 du disque d'anode, afin que l'autre moitié de la hauteur H2 de la tranche 16 puisse constituer la première cible 20 en molybdène.

Bien entendu le disque d'anode 9 peut être réalisé en un matériau autre que le molybdène, en graphite par exemple : dans ce cas, la seconde cible 21 est formée par le dépôt d'une couche 23 de rhodium comme dans l'exemple précédent, et la première cible 20 peut être également constituée par le dépôt d'une couche (non représentée) de molybdène.

Afin de permettre un bombardement électronique de l'une ou l'autre des deux cibles 20, 21, la cathode 8 comporte un premier et un second filament 30, 31 disposés respectivement en face de la première et de la seconde cibles 20, 21. En fonctionnement c'est l'un ou l'autre du premier ou du second filaments 30, 31 qui produit un faisceau d'électrons 32, 33. Le premier faisceau d'électrons 32 produit par le premier filament 30 bombarde la première cible 20 en molybdène sur une zone 35 qui constitue la source d'un rayonnement X dont les caractéristiques sont liées à celles du molybdène. Le second faisceau d'électrons 33, produit par le second filament 31, bombarde la seconde cible 21 en rhodium, sur une seconde zone 36 qui constitue la source d'un rayonnement X ayant des caractéristiques liées aux caractéristiques du rhodium.

Le choix de la cible 20, 21 bombardée s'opère ainsi par le choix du filament 30, 31 qui est mis en fonctionnement. Dans l'exemple non limitatif décrit, ce choix s'effectue en appliquant une basse tension de chauffage à l'un ou à l'autre des deux filaments 30, 31, par l'intermédiaire de transformateurs d'isolement 41, 42 ayant respectivement un premier et un second enroulement primaire 43, 44 et un premier et second enroulement secondaire 45, 46. Une source basse tension alternative 40 délivre une basse tension de chauffage de filament par un premier et un second plot 47, 48. Le premier plot 47 est relié simultanément à une première extrémité 50 de chacun des enroulements primaires 43, 44. La seconde borne 48 de la source basse tension 40 est reliée à un contact mobile 52 d'un circuit inverseur 53 à deux positions P1, P2 ; la seconde extrémité 55 du premier primaire 43 étant reliée au contact correspondant à la première position P1, et la seconde extrémité 56 du second enroulement primaire 44 étant reliée au contact correspondant à la seconde position P2. Une première extrémité 60 du premier enroulement secondaire 45 est reliée à une première extrémité 61 du premier filament 30, et une première extrémité 63 du second enroulement secondaire 46 est reliée à une première extrémité 62 du second filament 31. Les secondes extrémités 64 du premier et du second enroulement secondaire sont réunies et forment un point commun 70 qui est relié aux secondes extrémités 66 du premier et du second filament 30, 31.

D'autre part, le point 70 commun aux deux filaments 30, 31 et aux deux enroulements secondaires 45, 46 est relié à la polarité négative -HT de la haute tension d'alimentation du tube à rayons X, qui est fournie par un générateur haute tension 75. Le générateur haute tension 75 délivre la polarité négative -HT de la haute tension par une borne 76 et délivre la polarité positive +HT de cette haute tension par une seconde borne 77. La seconde borne 77 est reliée à l'extrémité 13 de l'arbre support métallique 12 qui porte le rotor 11, de sorte que la polarité positive +HT de la haute tension est appliquée à l'anode 9 par l'intermédiare de l'arbre support 12, du rotor 11, et de l'axe support métallique 15 disposé entre le rotor 11 et l'anode 9.

Dans ces conditions, la polarité positive de la haute tension étant reliée à l'anode 9, et la polarité négative étant reliée au premier et au second filament 30, 31 c'est-à-dire à la cathode 8, il suffit de mettre en marche la source basse tension 40 et de sélectionner avec le circuit inverseur 53 la position P1 pour chauffer le premier filament 30, et de mettre en marche le générateur haute tension 75 pour produire le premier faisceau d'électrons 32 qui bombarde la première cible 20 en molybdène, pour obtenir un rayonnement X dont le spectre comprend les raies caractéristiques du molybdène ; avec la sélection de la position P2, c'est le chauffage du second filament 31 qui est réalisé d'où il résulte que la première cible 20 en molybdène n'est plus bombardée que c'est la seconde cible 21 en rhodium qui est bombardée par le second faisceau d'électrons 33, de sorte que le spectre du rayonnement X qui est alors obtenu comporte des raies caractéristiques du rhodium.

Bien entendu, le générateur haute tension 75 délivre une haute tension dont la valeur peut être ajustée, selon que c'est la première cible 20 en molybdène ou la seconde cible 21 en rhodium qui est bombardée : la haute tension peut être réglée par exemple à environ 30 KV pour la cible en molybdène, et à environ 35 KV pour la cible en rhodium, bien entendu d'autres valeurs peuvent être choisies par l'homme du métier.

Il doit être noté que dans l'esprit de l'invention, le choix entre la première ou la seconde cible 20, 21 peut être réalisé de différentes manières à l'aide de deux cathodes entièrement indépendantes par exemple ou encore en produisant un unique faisceau d'électrons et à l'aide de moyens de déviation en eux-même classiques permettant de modifier la trajectoire du faisceau pour bombarder l'une ou l'autre des deux cibles.

Dans l'exemple non limitatif décrit, le rayonnement X produit à partir de la première ou de la seconde cible 20, 21 est émis dans toutes les directions, et il sort du tube à rayons X 6 par une première fenêtre 80, et sort de la gaîne 5 par une seconde fenêtre 81, les fenêtres 80, 81 étant orientées vers un plateau d'examen 85 et un dispositif récepteur de rayonnement X 86 situé sous le plateau d'examen 85. Le plateau d'examen 85 est destiné à porter un sein (non représenté d'une patiente), et le dispositif récepteur 86 est destiné par exemple à contenir, de manière classique, un couple film/écran renforçateur (non représenté).

Le bras support 3 comporte un trou 83 en vis-à-vis de la seconde fenêtre de sortie 81, pour le passage du rayonnement X, et il supporte, à l'opposé de la gaîne 5, un dispositif de filtration 89 ; dans l'exemple non limitatif décrit, le dispositif de filtration 89 est porté par l'intermédiaire d'une cale 90 permettant de rattraper une inclinaison de la gaîne 5. Le dispositif de filtration 89 porte lui-même un diaphragme 91 qui confère au rayonnement X des limites 92, 93, le rayonnement X formant alors un faisceau de rayons X utile 95 ayant les caractéristiques nécessaires à l'examen radiologique d'un sein.

La filtration du rayonnement X s'opère en interposant sur le trajet de ce dernier (symbolisé par une flèche 96 en traits pointillés) un filtre en molybdène si la cible bombardée est la première cible 20 en molybdène, ou un filtre en rhodium si c'est la seconde cible 21 en rhodium qui est bombardée. A cet effet, le dispositif de filtration 89 comporte par exemple des glissières 100 dans lesquelles est engagée et peut coulisser une monture 101, portant un premier filtre 102 en molybdène et un second filtre 103 en rhodium disposés l'un derrière l'autre ; le premier ou le second filtre 102, 103 étant interposé sur le trajet 96 du rayonnement X par un déplacement de la monture dans la glissière.

La filtration du rayonnement X produit à partir de la seconde cible 21 en rhodium, à l'aide d'un filtre en rhodium, s'opère selon les mêmes principes que la filtration classique par du molybdène d'un rayonnement X produit à partir d'une cible en molybdène.

Ainsi, en choisissant de bombarder électroniquement la seconde cible 21 en rhodium, et en filtrant le rayonnement X ainsi obtenu à l'aide du second filtre 103 en rhodium, on obtient un faisceau de rayons X utile 95 ayant un spectre en énergie relativement étroit dont l'énergie moyenne est de l'ordre de 20 KeV, et qui comprend les raies K alpha et K béta du rhodium. Il est à noter que la filtration du rayonnement X produit à partir de la seconde cible 21 en rhodium, peut être réalisée également à l'aide d'un matériau autre que le rhodium mais ayant un numéro atomique proche de celui du rhodium.

L'examen radiologique d'un sein à l'aide de la première cible 20 en mobyldène et du premiere filtre 102 en molybdène est particulièrement indiqué pour les seins d'épaisseur petite à moyenne et/ou de densité faible à moyenne, alors que l'utilisation de la seconde cible 21 en rhodium et du second filtre 103 en rhodium s'applique particulièrement à l'examen radiologique de seins ayant une épaisseur plus grande que la moyenne et/ou de densité plus élevée que la moyenne. Pour de tels seins ayant une absorption au rayonnement X plus élevée que la moyenne, le rayonnement X produit à partir du rhodium et filtré par du rhodium, permet d'obtenir une image au moins aussi bonne que celle qui est obtenue dans l'art antérieur à partir d'une cible en molybdène et à l'aide d'un filtre molybdène. En effet, bien que le rayonnement X obtenu à partir d'une cible en molybdène présente, pour la mammographie, des qualités supérieures à celles du rayonnement X obtenu à partir d'une cible en rhodium, l'utilisation de ce dernier rayonnement X présente l'avantage de ne pas exiger d'augmentation notable du temps de pose pour les seins à plus grande absorption, et par suite pour de tels seins, il permet d'améliorer la qualité de l'image par une diminution du flou cinétique, et il permet d'autre part d'éviter à la patiente l'absorption d'une dose importante de rayonnement, compte tenu de l'écart à la loi de réciprocité comme il a été précédemment mentionné dans le préambule. Il est à noter en outre que par rapport à la technique utilisant la cible et le filtre en molybdène, le rayonnement X obtenu par la cible en rhodium et filtré rhodium possède une énergie moyenne supérieure, de sorte qu'indépendamment du problème posé par l'écart à la loi de réciprocité présenté par le couple film/écran renforçateur, une plus grande quantité de rayons X traverse le sein, et la dose absorbée par la patiente est diminuée. Il est à signaler en outre que la plus grande énergie du rayonnement X permet d'utiliser un récepteur autre que le couple film/écran renforçateur et par exemple de réaliser des clichés du sein en technique dite xéromammographie.

Cette configuration de l'anode 9 qui comporte une première cible en molybdène et une seconde cible en rhodium est particulièrement intéressante en ce qu'elle permet, avec le même tube à rayons X, d'effectuer des examens radiologiques aussi bien pour des seins moins absorbants du rayonnement X, que plus absorbants du rayonnement X que la moyenne. Le choix d'utiliser la première ou la seconde cible 20, 21 et le premier et le second filtre 102, 103 peut être laissé au manipulateur ou au médecin, et être effectué d'une manière manuelle ; mais bien entendu ce choix peut également être réalisé de façon automatique, après une mesure de test par exemple. Il est à noter que cette configuration présente en outre l'avantage d'utiliser le même tube de rayons X 6 pour la réalisation de clichés en technique xéromammographique, en utilisant la seconde cible 21 en rhodium.

## Revendications

1. Dispositif de mammographie comportant un tube radiogène (6) produisant un faisceau de rayons X (95), le tube radiogène comportant une anode (9) et des moyens (8,30,31) pour produire au moins un faisceau d'électrons 33), caractérisé en ce que:
- l'anode du tube comporte deux cibles, l'une en rhodium (21), l'autre en molybdène(20), le rayonnement X produit à partir de la première ou la deuxième cible étant émis dans toutes les directions et sortant du tube par des fenêtres orientées vers un plateau d'examen (85);
- en ce qu'il comporte un dispositif de filtration (89) muni d'un diaphragme pour le passage du faisceau de rayons X, un filtre (103) en rhodium ou en un matériau ayant un numéro atomique proche du rhodium interposé sur le trajet (96) du faisceau de rayons X émis par la cible en rhodium et un filtre en molybdène (102) interposé sur le trajet (96) du faisceau de rayons (X) émis par la cible en molybdène, ces filtres pouvant être placés alternativement au dessus du diaphragme en fonction de la cible qui émet; et
- en ce que l'ensemble tube et dispositif de filtration étant porté par un bras (3), le tube étant incliné par rapport au dispositif de filtration de manière à ce que le rayonnement X utilisé soit pris dans les limites (92,93) du faisceau définies par le diaphragme et de manière à ce que le maximum d'intensité du rayonnement se trouve autour d'un seul axe (96), quelle que soit la cible qui émet.

2. Dispositif de mammographie selon la revendication 1, caractérisé en ce que les moyens (8,30,31) pour produire au moins un faisceau d'électrons comporte une cathode (8) ayant un premier et un second filament(30,31), le premier filament produisant un premier faisceau d'électrons (32) destiné à bombarder la cible en molybdène (20) et une second filament (31) produisant un second faisceau d'électrons (33) destiné à bombarder la cible en rhodium (21).

3. Dispositif de mammographie selon la revendication 2, caractérisé en ce qu'il comporte un moyen de commutation (53) permettant de sélectionner le fonctionnement du premier filament (30) ou le fonctionnement du second filament (31).

4. Dispositif de mammographie selon l'une des revendications précédentes, caractérisé en ce que l'anode (9) est une anode tournante.

5. Dispositif de mammographie selon l'une des revendications précédentes, caractérisé en ce que la cible en rhodium (21) est formée par une couche (23) de rhodium déposée sur l'anode (9).

6. Dispositif de mammographie selon la revendication 1, caractérisé en ce que la cible en molybdène (20) est directement formée par l'anode (9) qui est constituée en molybdène.

7. Dispositif de mammographie selon la revendication 1, caractérisé en ce que cible en rhodium (21) et la cible en molybdène (20) sont formées sur une tranche (16) de l'anode (9).

## Claims

1. A mammography device comprising a radiogenic tube producing an X-ray beam (95), the radiogenic tube comprising an anode (9) and means (8, 30, 31) to produce at least one electron beam (33); wherein:
- the anode of the tube has two targets, one made of rhodium (21) and the other made of molybdenum (20), the radiation produced from the first target or the second target being emitted in every direction and emerging from the tube through windows oriented towards an examination table (85);
- said device comprises a filtering device (89) provided with a diaphragm for the passage of the X-ray beam, a filter (103) made of rhodium or made of a material having an atomic number close to that of rhodium, interposed in the path of the X-ray beam emitted by the target made of rhodium, and a filter made of molybdenum (102), interposed on the path (96) of the X-ray beam emitted by the target made of molybdenum, it being possible for these filters to be placed alternately above the diaphragm as a function of the target that is emitting; and
- the assembly formed by the tube and the filtering device being borne by an arm (3), the tube is tilted in relation to the filtering device so that the X-radiation used is contained within the limits (92, 93) of the beam defined by the diaphragm and so that the maximum intensity of the radiation is located around only one axis (96), whatever may be the target that is emitting.

2. A mammography device according to claim 1, wherein the means (8, 30, 31) for producing at least one electron beam comprise a cathode (8) having a first filament and a second filament (30, 31), the first filament producing a first electron beam (32) designed to bombard the molybdenum target (20) and the second filament (31) producing a second electron beam (33) designed to bombard the rhodium target (21).

3. A mammography device according to claim 2, comprising a switch-over means (53) enabling the selection of the working of the first filament (30) or of the working of the second filament (31).

4. A mammography device according to any of the foregoing claims, wherein the anode (9) is a rotating anode.

5. A mammography device according to any of the foregoing claims, wherein the rhodium target (21) is formed by a layer (23) of rhodium deposited on the anode (9).

6. A mammography device according to claim 1, wherein the molybdenum target (9) is directly formed by the anode (9) which is made of molybdenum.

7. A mammography device according to claim 1, wherein the rhodium target (21) and the molybdenum target (20) are formed on an edge (16) of the anode (9).

## Patentansprüche

1. Mammographiegerät, mit einer Röntgenröhre (6), die ein Röntgenstrahlbündel (95) erzeugt, wobei die Röntgenröhre eine Anode (9) und eine Anordnung (8, 30, 31) aufweist zur Erzeugung wenigstens eines Elektronenstrahlbündels (33), dadurch gekennzeichnet, daß:
- die Anode der Röhre zwei Targets enthält, von denen das eine aus Rhodium (21) und das andere aus Molybdän (20) besteht, wobei die von dem ersten oder dem zweiten Target erzeugte Röntgenstrahlung in alle Richtungen ausgesandt wird und aus der Röhre durch Fenster austritt, die zu einer Untersuchungsplatte (85) hin gerichtet sind;
- es eine Filteranordnung (89) aufweist, die mit einer Blende zum Durchlaß des Röntgenstrahlbündels versehen ist, einen Filter (103) aus Rhodium oder aus einem Material, dessen Ordnungszahl in der Nähe derjenigen von Rhodium liegt, aufweist, der in den Weg (96) des Röntgenstrahlbündels eingesetzt wird, welches vom Target aus Rhodium ausgesandt wird und ein Filter aus Molybdän (102) aufweist, der in den Weg (96) des Röntgenstrahlbündels eingesetzt wird, welches vom Target aus Molybdän ausgesandt wird, wobei diese Filter abwechselnd oberhalb der Blende als Funktion des aussendenden Targets plaziert werden können und
- die Gesamtheit aus Röhre und Filteranordnung von einem Arm (3) getragen wird, wobei die Röhre bezüglich der Filteranordnung derart geneigt ist, daß die verwendete Röntgenstrahlung eingesetzt wird innerhalb der Begrenzungen (92, 93) des Bündels, die durch die Blende vorgegeben sind und derart, daß das Intensitätsmaximum der Strahlung um eine einzige Achse (96) herum ausgebildet ist, unabhängig von dem verwendeten Target.

2. Mammographiegerät nach Anspruch 1, dadurch gekennzeichnet, daß die Anordnung (8, 30, 31) zur Erzeugung wenigstens eines Elektronenstrahlbündels eine Kathode (8) aufweist, die mit einer ersten und einer zweiten Wendel (30, 31) versehen ist, wobei die erste Wendel ein erstes Elektronenstrahlbündel (32) erzeugt, das zur Bestrahlung eines Targets aus Molybdän (20) dient, während die zweite Wendel (31) ein zweites Elektronenstrahlbündel (33) erzeugt, das zur Bestrahlung des Targets aus Rhodium (21) dient.

3. Mammographiegerät nach Anspruch 2, dadurch gekennzeichnet, daß es eine Umschaltanordnung 53 aufweist, zur Auswahl des Betriebes der ersten Wendel (30) oder des Betriebes der zweiten Wendel (31).

4. Mammographiegerät nach einem der vorhergehenden Ansprüche, dadurch gekennzeichnet, daß die Anode (9) eine Drehanode ist.

5. Mammographiegerät nach einem der vorhergehenden Ansprüche, dadurch gekennzeichnet, daß das Target aus Rhodium (21) aus einer auf der Anode (9) aufgebrachten Rhodiumschicht (23) besteht.

6. Mammographiegerät nach Anspruch 1, dadurch gekennzeichnet, daß das Target aus Molybdän (20) direkt durch die Anode (9) gebildet wird, welche aus Molybdän besteht.

7. Mammographiegerät nach Anspruch 1, dadurch gekennzeichnet, daß das Target aus Rhodium (21) und das Target aus Molybdän (20) auf einem Teil (16) der Anode (9) ausgebildet sind.
